(19) Europäisches Patentamt

European Patent Office

Office européen des brevets

(11) **EP 0 490 380 B1**

(12) **EUROPEAN PATENT SPECIFICATION**

(45) Date of publication and mention
of the grant of the patent:
**18.02.1998 Bulletin 1998/08**

(21) Application number: **91121354.4**

(22) Date of filing: **12.12.1991**

(51) Int. Cl.$^6$: **A61K 31/13**
// (A61K33/24, 31:13),
(A61K31/475, 31:13),
(A61K31/71, 31:13),
(A61K31/70, 31:13)

(54) **Use of N,N'-bis 3-(ethylamino)propyl -1,7-heptanediamine and a cytotoxic agent**

Verwendung von N,N'-Bis 3-(ethylamino)propyl-1,7-Heptandiamin und einem zytotoxischen Mittel

Utilisation de N,N'-bis 3-(ethylamino)propyl-1,7-heptanediamine et un agent cytotoxique

(84) Designated Contracting States:
**AT BE CH DE DK ES FR GB GR IT LI LU NL SE**

(30) Priority: **13.12.1990 US 626814**

(43) Date of publication of application:
**17.06.1992 Bulletin 1992/25**

(73) Proprietor:
**MERRELL PHARMACEUTICALS INC.
Cincinnati, Ohio 45215-6300 (US)**

(72) Inventors:
• **Prakash, Nellikunja J.
Cincinnati, Ohio 45241 (US)**
• **Bowlin, Terry L.
Maineville, Ohio 45039 (US)**

(74) Representative:
**VOSSIUS & PARTNER
Postfach 86 07 67
81634 München (DE)**

(56) References cited:
**EP-A- 0 378 146        EP-A- 0 399 519**

• **ANTICANCER RESEARCH, vol. 10, no. 5A,
September-October 1990, pages 1281-1288; N.J.
PRAKASH et al.: "Antitumor activity of a novel
synthetic polyamine analogue, N,N' -Bis-(3-
(ethylamino)-propyl)-1-7-heptane diamine:
potentiation by polyamine oxidase inhibitors"**

## Description

Neoplastic disease states in humans are recognized throughout the world as being serious and oftentimes life-threatening conditions. These neoplastic diseases, which are characterized by rapidly-proliferating cell growth, have been and continue to be the subject of worldwide research efforts directed toward the identification of therapeutic agents which are effective in the treatment of patients suffering therefrom. Effective therapeutic agents can be characterized as those which prolong the survivability of the patient, which inhibit the rapidly-proliferating cell growth associated with the neoplasm, or which effect a regression of the neoplasm. Research in this area is primarily focused toward identifying agents which would be therapeutically effective in humans. Typically, compounds are tested for antineoplastic activity in small mammals, such as mice, in experiments designed to be predictive of antineoplastic activity not only in those animals but also in humans against specific neoplastic disease states.

Certain vinca alkaloids, antibiotics, antimetabolites and platinum coordination complexes are well known as effective antineoplastic agents [See Calabresi, P., and Chabner, B. A., "CHEMOTHERAPY OF NEOPLASTIC DISEASES", Section XII, GOODMAN AND GILLMAN'S, THE PHARMACOLOGICAL BASIS OF THERAPEUTICS, 8th ed., 1990, Pergamon Press Inc., Elmsford, New York]. For example, vinblastine and vincristine are vinca alkaloids which are useful antineoplastic agents. Antibiotics which possess antineoplastic properties include adriamycin (doxorubicin), dactinomycin (actinomycin D), daunorubicin (daunomycin, rubidomycin), bleomycin, plicamycin (mithramycin) and mitomycin (mitomycin C). Methotrexate, cytarabine (AraC), azauridine, azaribine, fluorodeoxyuridine, deoxycoformycin and mercaptopurine are examples of antimetabolites with antineoplastic properties. Cisplatin (cis-DDP) and carboplatin are platinum coordination complexes which are useful antineoplastic agents. These agents are proven to be useful in the treatment of patients suffering from a variety of neoplastic disease states.

Certain polyamine compounds, such as N,N'-bis[3-(ethylamino)propyl]-1,7-heptanediamine, are also well known as effective antineoplastic agents [European Patent Application Publication No. 0 378 146, published July 18, 1990, and European Patent Application Publication No. 0 311 068, published April 12, 1989]. These polyamines are also useful in the treatment of patients suffering from a variety of neoplastic disease states.

It has now been found that in treating a patient afflicted with certain neoplastic disease states, conjunctive therapy with N,N'-bis[3-(ethylamino)propyl]-1,7-heptanediamine and a cytotoxic agent selected from the group consisting of an antineoplastic vinca alkaloid, an antineoplastic antibiotic, an antineoplastic antimetabolite and an antineoplastic platinum coordination complex, will provide a synergistic antineoplastic effect.

The present invention concerns the use of N,N'-bis[3-(ethylamino)propyl]-1,7-heptanediamine in conjunction with a cytotoxic agent selected from the group consisting of an antineoplastic vinca alkaloid, an antineoplastic antibiotic, an antineoplastic antimetabolite and an antineoplastic platinum coordination complex for the preparation of a pharmaceutical composition useful in treating a patient suffering from a neoplastic disease state.

More specifically, the present invention concerns the use of N,N'-bis[3-(ethylamino)propyl]-1,7-heptanediamine in conjunction with vinblastine, cisplatin, AraC or adriamycin for the preparation of a pharmaceutical composition useful in treating a patient suffering from a neoplastic disease state.

The polyamine N,N'-bis[3-(ethylamino)propyl]-1,7-heptanediamine can be prepared as described in European Patent Application Publication No. 0 378 146, published July 18, 1990, and European Patent Application Publication No. 0 311 068, published April 12, 1989. In order to illustrate the preparation of N,N'-bis[3-(ethylamino)propyl]-1,7-heptanediamine, the following example is provided. The example is illustrative only and is not intended to limit the invention in any way. All temperatures are in degrees Celsius and the following abbreviations are used: (g) is grams, (mol) is moles, (ml) is milliliters, (l) is liters, (TLC) is thin layer chromatography, (THF) is tetrahydrofuran, (DMF) is dimethylformamide, (mp) is melting point, (mm/Hg) is pressure expressed as millimeters of mercury, (bp) is boiling point.

## EXAMPLE 1

### N,N'-Bis[3-(ethylamino)propyl]-1,7-heptanediamine

**Steps A and B:** 1,5,13,17-Tetraazaheptadecane tetrahydrochloride
Prepare the title compound by the method of Israel et al., J. Med. Chem. 7, 710 (1964).

**Step C:** 1,5,13,17-Tetra(t-butoxycarbonyl)-1,5,13,17-tetraazaheptadecane
Combine 1,5,13,17-tetraazaheptadecane tetrahydrochloride (3.9 gm, 0.01 mol) and sodium hydroxide (1.76 gm, 0.44 mol) in water (44 ml) and stir until homogeneous. To this mixture add di-t-butyldicarbonate (9.6 gm, 0.044 mol) in THF (88 ml) and stir for 3 hours. Dilute the mixture with ethyl acetate (EtOAc) [300 ml] and separate the organic layer. Dry the organic layer over anhydrous $MgSO_4$ and evaporate *in vacuo* to obtain a viscous oil. Purify the residue by flash chromatography (silica gel) eluting with 25% EtOAc/hexane to yield 3.0 gm of the title compound. $R_f$ is 0.20 on silica gel plates eluted with 25% EtOAc/hexane.

**Step D:** 3,7,15,19-Tetra(t-butoxycarbonyl)-3,7,15,19-tetraazaheneicosane

Combine 1,5,13,17-tetra(t-butoxycarbonyl)-1,5,13,17-tetraazaheptadecane (3.0 gm, 0.0046 mol) and sodium hydride (50% in oil) [0.45 gm, 0.011 mol] in DMF (9 ml) and stir the mixture until hydrogen evolution ceases. Add ethyl iodide (0.9 ml, 0.011 mol) and stir the mixture for 18 hours. Evaporate the DMF *in vacuo* and partition the residue between ethyl acetate (600 ml) and water (200 ml). Separate the organic layer, dry the organic layer over anhydrous $MgSO_4$ and evaporate *in vacuo*. Purify the residue by flash chromatography (silica gel) eluting with 20% EtOAc/hexane to yield 1.68 gm of the title compound. $R_f$ is 0.5 on silica gel plates eluted with 25% EtOAc/hexane.

**Step E:** N,N'-Bis[3-(ethylamino)propyl]-1,7-heptanediamine

Treat 3,7,15,19-tetra(t-butoxycarbonyl)-3,7,15,19-tetraazaheneicosane (1.68 gm, 0.0024 mol) with HCl in methanol (50 ml, 1.0 N) and stir overnight. Filter the mixture and recrystallize the title compound from methanol/water (20:80, v/v) to yield 0.5 gm of the title compound. $R_f$ is 0.39 on silica gel plates eluted with 40% ammonia (concentrated) in methanol; mp 322-23°C with degradation.

The antineoplastic vinca alkaloids, such as vinblastine and vincristine, the antineoplastic antibiotics, such as adriamycin (doxorubicin), dactinomycin (actinomycin D), daunorubicin (daunomycin, rubidomycin), bleomycin, plicamycin (mithramycin) and mitomycin (mitomycin C), the antineoplastic antimetabolites, such as methotrexate, cytarabine (AraC), azauridine, azaribine, fluorodeoxyuridine, deoxycoformycin and mercaptopurine, and the antineoplastic platinum coordination complexes, such as cisplatin (cis-DDP) and carboplatin, are readily available and their use as antineoplastic agents is well known and appreciated in the art [For example, See Calabresi, P., and Chabner, B. A., "CHEMOTHERAPY OF NEOPLASTIC DISEASES", Section XII, GOODMAN AND GILLMAN'S, THE PHARMACOLOGICAL BASIS OF THERAPEUTICS, 8th ed., 1990, Pergamon Press Inc., Elmsford, New York].

The present invention concerns the use of N,N'-bis[3-(ethylamino)propyl]-1,7-heptanediamine and a cytotoxic agent selected from the group consisting of an antineoplastic vinca alkaloid, an antineoplastic antibiotic, an antineoplastic antimetabolite and an antineoplastic platinum coordination complex for the preparation of a pharmaceutical Composition useful in treating a patient suffering from a neoplastic disease state. This pharmaceutical composition prepared provides a conjunctive therapy with unexpectedly synergistic antineoplastic effect.

As used herein, the term "patient" refers to a warm-blooded animal such as a mammal which is afflicted with a neoplastic disease state. It is understood that dogs, cats, rats, mice, horses, bovine cattle, sheep, and humans are examples of animals within the scope of the meaning of the term.

The term "neoplastic disease state" as used herein refers to an abnormal state or condition characterized by rapidly proliferating cell growth or neoplasm. Neoplastic disease states for which conjunctive therapy according to the present invention will be particularly useful include: leukemias such as, but not limited to, acute lymphoblastic, chronic lymphocytic, acute myeloblastic and chronic myelocytic; carcinomas, such as, but not limited to, those of the cervix, oesophagus, stomach, small intestines, brain, colon and lungs; sarcomas, such as, but not limited to, oesteroma, osteosarcoma, lipoma, liposarcoma, hemangioma and hemangiosarcoma; melanomas, including amelanotic and melanotic; and mixed types of neoplasias such as, but not limited to carcinosarcoma, lymphoid tissue type, follicular reticulum, cell sarcoma and Hodgkins Disease. Of course, one skilled in the art will recognize that not every combination of conjunctive therapy according to the present invention will be equally effective against each of the neoplastic disease states. Selection of the most appropriate combination is within the ability of one of ordinary skill in the art and will depend on a variety of factors including assessment of results obtained in standard animal cancer models and the effectiveness of the individual agents as monotherapy in treating particular neoplastic disease states.

For example, conjunctive therapy with N,N'-bis[3-(ethylamino)propyl]-1,7-heptanediamine and vinblastine will be particularly effective in the treatment of a patient afflicted with leukemia, carcinoma, lymphoma or osteosarcoma. Conjunctive therapy with N,N'-bis[3-(ethylamino)propyl]-1,7-heptanediamine and cisplatin will be particularly effective in the treatment of a patient afflicted with carcinoma, testicular teratoma or ovarian carcinoma. Conjunctive therapy with N,N'-bis[3-(ethylamino)propyl]-1,7-heptanediamine and adriamycin will be particularly effective in the treatment of a patient afflicted with breast carcinoma, leukemia, lymphoma and ovarian carcinoma. Conjunctive therapy with N,N'-bis[3-(ethylamino)propyl]-1,7-heptanediamine and AraC will be particularly effective in the treatment of a patient afflicted with leukemia.

The polyamine compound N,N'-bis[3-(ethylamino)propyl]-1,7-heptanediamine in conjunction with a cytotoxic agent selected from the group consisting of an antineoplastic vinca alkaloid, an antineoplastic antibiotic, an antineoplastic antimetabolite and an antineoplastic platinum coordination complex is used for the preparation of a pharmaceutical composition in the conjunctive therapy of a patient afflicted with a neoplastic disease state as described above. As used herein, the term "conjunctive therapy" contemplates co-administration of N,N'-bis[3-(ethylamino)propyl]-1,7-heptanediamine along with the cytotoxic agent. This co-administration may take place at essentially the same time, it may take place sequentially, or it may take place alternately.

In providing co-administration at essentially the same time, the courses of treatment with the polyamine and the selected cytotoxic agent run essentially concomitantly. In providing sequential co-administration, a full course of treatment of one of the agents is terminated and then followed by a full course of treatment of the other. In providing alternate co-administration, a partial course of treatment of one of the agents is terminated and then followed by a partial course of treatment of the other in an alternating manner until a full treatment of each agent is administered. When the polyamine compound and the selected cytotoxic agent are co-administered in a sequential or an alternate manner, it is generally preferred to administer the cytotoxic agent first and the polyamine compound last.

In effecting the conjunctive therapy by using the pharmaceutical composition prepared according to the present invention, it is preferred to co-administer the polyamine compound and the selected cytotoxic agent in a sequential or an alternate manner. It is most preferred to co-administer the polyamine compound and the selected cytotoxic agent in a sequential manner.

As used herein, the term "effective antineoplastic amount" refers to an amount which is effective, upon single or multiple dose administration to the patient, in controlling the growth of the neoplasm or in prolonging the survivability of the patient beyond that expected in the absence of such treatment. As used herein, "controlling the growth" of the neoplasm refers to slowing, interrupting, arresting or stopping its growth and does not necessarily indicate a total elimination of the neoplasm.

An effective antineoplastic amount of N,N'-bis[3-(ethylamino)propyl]-1,7-heptanediamine is expected to vary from about 10 milligram per kilogram of body weight per day (mg/kg/day) to about 100 mg/kg/day and preferably will be about 5 mg/kg/day to about 50 mg/kg/day.

The effective antineoplastic amounts of the various cytotoxic agents are well known and appreciated in the art. For example, an effective antineoplastic amount of vinblastine is expected to vary from about 3 $mg/m^2$/day to about 10 $mg/m^2$/day. An effective antineoplastic amount of cisplatin is expected to vary from about 20 $mg/m^2$/day to about 50 $mg/m^2$/day. An effective antineoplastic amount of adriamycin is expected to vary from about 60 $mg/m^2$/day to about 70 $mg/m^2$/day. An effective antineoplastic amount of AraC is expected to vary from about 1 $mg/m^2$/day to about 200 $mg/m^2$/day.

In effecting treatment of a patient afflicted with a disease state described above, the pharmaceutical composition containing N,N'-bis[3-(ethylamino)propyl]-1,7-heptanediamine can be administered in any form or mode which makes the compound bioavailable in effective amounts, including oral and parenteral routes. For example, it can be administered orally, subcutaneously, intramuscularly, intravenously, transdermally, intranasally, rectally, and the like. Oral administration is generally preferred. One skilled in the art of preparing formulations can readily select the proper form and mode of administration depending upon the particular circumstances, including the disease state to be treated, the stage of the disease, the form of administration of the selected cytotoxic agent, the manner of co-administration selected, and the like.

The pharmaceutical composition containing the compound N,N'-bis[3-(ethylamino)propyl]-1,7-heptanediamine can be administered alone or in combination with pharmaceutically acceptable carriers or excipients, the proportion and nature of which are determined by the solubility and chemical properties of N,N'-bis[3-(ethylamino) propyl]-1,7-heptanediamine, the chosen route of administration, and standard pharmaceutical practice.

The compound N,N'-bis[3-(ethylamino)propyl]-1,7-heptanediamine contained in the pharmaceutical composition, while effective itself, may be formulated and administered in the form of its pharmaceutically acceptable acid addition salt for purposes of stability, convenience of crystallization, increased solubility and the like.

The selected cytotoxic agent can be administered in a manner as is well known and accepted for the particular agent. For example, vinblastine, cisplatin, adriamycin and AraC are administered intravenously.

The following examples are provided in order to illustrate the method of use of the present invention. These examples are intended to be illustrative only and are not to be construed to limit the scope of the invention in any way.

## EXAMPLE 2

Conjunctive Therapy in the Treatment of L1210 Leukemia *in vivo*

Groups of 5 mice (BDF1 male) were inoculated i.p. with $10^5$ L1210 leukemia cells on day 0. Vinblastine was administered at 0.5 mg/kg, i.p., on day 3. The compound N,N'-bis(3-(ethylamino) propyl]-1,7-heptanediamine was administered at 5 mg/kg, every 3 hours, 4 times per day, on days 4 and 5. Control group received no treatment after inoculation. Relative survival time was determined and expressed as % T/C (mean survival time treated/mean survival time control x 100). The results of these studies are presented in Table 1.

Table 1

| Antitumor Activity against L1210 Leukemia | | |
|---|---|---|
| Group | Dose, mg/Kg/day (Days Dosed) | Mean Survival Time (days±S.D.[a]) |
| Control | - | 7.6±0.5 |
| Vinblastine | 0.5 (day 3) | 10.0±0.7 |
| 28,314[b] | 20 (days 4,5) | 12.6±0.9 |
| Vinblastine + 28,314 | 20 (days 4,5) | 100% Cured[c] |

[a]S.D. = Standard Deviation
[b]28,314 = N,N'-Bis[3-(ethylamino)propyl]-1,7-heptanediamine
[c]Animals are considered cured if they survive beyond 45 days

## EXAMPLE 3

Effect of Conjunctive Therapy in Mammalian Cell Culture

MCA38 (mouse colon adenocarcinoma) and HeLa cells were grown in cell culture in the presence or absence of various concentrations of N,N'-Bis[3-(ethylamino)propyl]-1,7-heptanediamine (Compound 28,314), cisplatin, adriamycin, vinblastine and cytarabine (AraC) as indicated in Tables 2-9. Viablity of the cells after a 96 hour incubation was determined by a colorimetric assay, essentially as described by Carmichael et al. [*Cancer Res.* 47, 936 (1987)], whereby the cellular reduction of MTT [3-(4,5-dimethylthiazol-2-yl)-2,5-diphenyltetrazolium bromide] is measured.

$IC_{50}$ values were calculated for the individual treatments as well as for the combined treatments of Compound 28,314 with the various other agents. The $IC_{50}$ values at the various concentrations of test agents are presented in Tables 2-9.

In addition, the Fractional Inhibitory Concentration (FIC) for the various treatments was calculated. FIC's are an accepted measurement of synergy [Middleton and Westmacott, *J.Antimicrobial Chemother.* 11, 427 (1983)] and represents the $IC_{50}$ of a drug in combination divided by the $IC_{50}$ of the drug acting alone. For two interacting drugs, the sum of the FIC's of each of the treatments expresses the extent of the synergistic interaction. The FIC may be computed for any point on the isobole (usually the most significant is computed) by the following calculation:

$$FIC = [IC_{50} \text{ Compound A Combined}/IC_{50} \text{ Compound A Alone}] + [IC_{50} \text{ Compound B Combined}/IC_{50}$$

$$\text{Compound B Alone}].$$

Where FIC is less than 1, there is synergy between the two treatments, where FIC is 1, there is no synergy (additive effect). The smaller the value for FIC, the greater the synergistic interaction. The calculated values for FIC at the indicated concentrations are shown in Tables 2-9.

This data shows the synergistic antineoplastic effect of conjunctive therapy with Compound 28,314 and cytotoxic agents such as cisplatin, adriamycin, AraC, and vinblastine. However, in Table 7, an additive effect only was found for the conjunctive treatment of HeLa cells with Compound 28,314 and adriamycin.

Table 2

| MCA 38 Cells Treated with Compound 28,314[a] and Cisplatin | |
|---|---|
| Concentration of 28,314 ($\mu$g/mL) | IC$_{50}$ ($\mu$g/mL) Cisplatin |
| 0 | 0.847 |
| 0.2 | 0.58 |
| 0.39 | 0.387 |
| 0.78 | 0.376 |
| 1.56 | 0.276 |
| 3.125* | 0.177* |
| 6.25 | 0.165 |
| 12.5 | 0.053 |
| 17.7[b] | 0 |
| *FIC | 0.39 (synergism) |

[a]28,314 = N,N'-Bis[3-(ethylamino)propyl]-1,7-heptanediamine
[b]IC$_{50}$ 28,314 alone

Table 3

| MCA 38 Cells Treated with Compound 28,314[a] and Adriamycin | |
|---|---|
| Concentration of 28,314 ($\mu$g/mL) | IC$_{50}$ ($\mu$g/mL) Adriamycin |
| 0 | 0.0079 |
| 0.39 | 0.0075 |
| 0.78 | 0.0073 |
| 1.563 | 0.0070 |
| 3.125 | 0.0063 |
| 6.25* | 0.0025* |
| 12.5 | 0.0018 |
| 25[b] | 0 |
| *FIC | 0.57 (synergism) |

[a]28,314 = N,N'-Bis[3-(ethylamino)propyl]-1,7-heptanediamine
[b]IC$_{50}$ 28,314 alone

Table 4

| MCA 38 Cells Treated with Compound 28,314[a] and AraC | |
|---|---|
| Concentration of 28,314 ($\mu$g/mL) | IC$_{50}$ ($\mu$g/mL) AraC |
| 0 | 0.66 |
| 0.1563 | 0.596 |
| 0.3125 | 0.488 |
| 0.625 | 0.36 |
| 1.25* | 0.23* |
| 2.5 | 0.071 |
| 3.54[b] | 0 |
| *FIC | 0.70 (synergism) |

[a]28,314 = N,N'-Bis[3-(ethylamino) propyl]-1,7-heptanediamine
[b]IC$_{50}$ 28,314 alone

Table 5

| MCA 38 Cells Treated with Compound 28,314[a] and Vinblastine | |
|---|---|
| Concentration of 28,314($\mu$g/mL) | IC$_{50}$ ($\mu$g/mL) Vinblastin |
| 0 | 0.0595 |
| 0.1563 | 0.0548 |
| 0.3125 | 0.043 |
| 0.625 | 0.033 |
| 1.25* | 0.025* |
| 2.5 | 0.015 |
| 5.0 | 0.003 |
| 10.0[b] | 0 |
| *FIC | 0.55 (synergism) |

[a]28,314 = N,N'-Bis[3-(ethylamino) propyl]-1,7-heptanediamine
[b]IC$_{50}$ 28,314 alone

Table 6

| HeLa Cells Treated with Compound 28,314[a] and Cisplatin | |
|---|---|
| Concentration of 28,314($\mu$g/mL) | IC$_{50}$ ($\mu$g/mL) Cisplatin |
| 0 | 0.359 |
| 0.39 | 0.336 |
| 0.78 | 0.276 |
| 1.563 | 0.186 |
| 3.125 | 0.130 |
| 6.25 | 0.088 |
| 12.5* | 0.074* |
| 25 | 0.068 |
| 50 | 0.068 |
| 100 | 0.057 |
| ~200[b] | 0 |
| *FIC | 0.27 (synergism) |

[a]28,314 = N,N'-Bis[3-(ethylamino)propyl]-1,7-heptanediamine
[b]IC$_{50}$ 28,314 alone

Table 7

| HeLa Cells Treated with Compound 28,314[a] and Adriamycin | |
|---|---|
| Concentration of 28,314($\mu$g/mL) | IC$_{50}$ ($\mu$g/mL) Adriamycin |
| 0 | 0.0086 |
| 0.2 | 0.0067 |
| 0.39* | 0.0058* |
| 0.78 | 0.0048 |
| 1.5625 | 0.0015 |
| 1.59[b] | 0 |
| *FIC | 0.92 (additive) |

[a]28,314 = N,N'-Bis[3-(ethylamino)propyl]-1,7-heptanediamine
[b]IC$_{50}$ 28,314 alone

Table 8

| Hela Cells Treated with Compound 28,314[a] and AraC | |
|---|---|
| Concentration of 28,314 ($\mu$g/mL) | IC$_{50}$ ($\mu$g/mL) AraC |
| 0 | 1.02 |
| 0.1563 | 0.63 |
| 0.3125 | 0.60 |
| 0.625 | 0.53 |
| 1.25 | 0.37 |
| 2.5 | 0.35 |
| 5 | 0.22 |
| 10* | 0.15* |
| 20 | 0.14 |
| 40 | 0.14 |
| 80 | 0.14 |
| ~160 | 0 |
| *FIC | 0.21 (synergism) |

[a]28,314 = N,N'-Bis[3-(ethylamino) propyl]-1,7-heptanediamine
[b]IC$_{50}$ 28,314 alone

Table 9

| HeLa Cells Treated with Compound 28,314[a] and Vinblastine | |
|---|---|
| Concentration of 28,314 ($\mu$g/mL) | IC$_{50}$ ($\mu$g/mL) Vinblastin |
| 0 | 0.0016 |
| 0.1563 | 0.00133 |
| 0.3125 | 0.00133 |
| 0.625 | 0.00119 |
| 1.25 | 0.00117 |
| 2.5 | 0.00063 |
| 5* | 0.00024* |
| 10 | 0.00011 |
| 20 | 0.00009 |
| *FIC | 0.31 (synergism) |

[a]28,314 = N,N'-Bis[3-(ethylamino) propyl]-1,7-heptanediamine
[b]IC$_{50}$ 28,314 alone

**Claims**

**Claims for the following Contracting States : AT, BE, CH, DE, DK, FR, GB, IT, LI, LU, NL, SE**

1. Use of N,N'-bis[3-(ethylamino)propyl]-1,7-heptanediamine and of a cytotoxic agent selected from the group consisting of an antineoplastic vinca alkaloid, an antineoplastic antibiotic, an antineoplastic antimetabolite and an antineoplastic platinum coordination complex for the preparation of pharmaceutical compositions useful in the conjunctive therapy of a patient suffering from a neoplastic disease state.

2. Use according to Claim 1 wherein the cytotoxic agent is vinblastine.

3. Use according to Claim 1 wherein the cytotoxic agent is cisplatin.

4. Use according to Claim 1 wherein the cytotoxic agent is adriamycin.

5. Use according to Claim 1 wherein the cytotoxic agent is cytarabine.

6. Use according to Claim 1 wherein the neoplastic disease state is a leukemia.

7. Use according to Claim 1 wherein the neoplastic disease state is a carcinoma.

**Claims for the following Contracting States : ES, GR**

1. A method for the preparation of pharmaceutical compositions or a kit useful in the conjunctive therapy of a patient suffering from a neoplastic disease state comprising combining N,N'-bis[3-(ethylamino)propyl]-1,7-heptanediamine and a cytotoxic agent selected from the group consisting of an antineoplastic vinca alkaloid, an antineoplastic antibiotic, an antineoplastic antimetabolite and an antineoplastic platinum coordination complex with a pharmaceutically acceptable carrier.

2. A method according to Claim 1 wherein the cytotoxic agent is vinblastine.

3. A method according to Claim 1 wherein the cytotoxic agent is cisplatin.

4. A method according to Claim 1 wherein the cytotoxic agent is adriamycin.

5. A method according to Claim 1 wherein the cytotoxic agent is cytarabine.

6. A method according to Claim 1 wherein the neoplastic disease state is a leukemia.

7. A method according to Claim 1 wherein the neoplastic disease state is a carcinoma.

**Patentansprüche**

**Patentansprüche für folgende Vertragsstaaten : AT, BE, CH, DE, DK, FR, GB, IT, LI, LU, NL, SE**

1. Verwendung von N,N'-Bis[3-(ethylamino)propyl]-1,7-Heptandiamin und einem cytotoxischen Mittel, ausgewählt aus einem antineoplastischen Vincaalkaloid, einem antineoplastischen Antibiotikum, einem antineoplastischen Antimetaboliten und einem antineoplastischen Platinkoordinationskomplex, zur Herstellung eines Arzneimittels, das für eine Kombinationstherapie eines Patienten, der an einem neoplastischen Krankheitszustand leidet, geeignet ist.

2. Verwendung nach Anspruch 1, wobei es sich bei dem cytotoxischen Mittel um Vinblastin handelt.

3. Verwendung nach Anspruch 1, wobei es sich bei dem cytotoxischen Mittel um Cisplatin handelt.

4. Verwendung nach Anspruch 1, wobei es sich bei dem cytotoxischen Mittel um Adriamycin handelt.

5. Verwendung nach Anspruch 1, wobei es sich bei dem cytotoxischen Mittel um Cytarabin handelt.

6. Verwendung nach Anspruch 1, wobei es sich bei dem neoplastischen Krankheitszustand um eine Leukämie handelt.

7. Verwendung nach Anspruch 1, wobei es sich bei dem neoplastischen Krankheitszustand um ein Karzinom handelt.

**Patentansprüche für folgende Vertragsstaaten : ES, GR**

1. Verfahren zur Herstellung von Arzneimitteln oder einer Arzneimittelzusammenstellung, die für die Kombinationstherapie eines Patienten geeignet ist, der an einem neoplastischen Krankheitszustand leidet, umfassend die Kombination von N,N'-Bis[3-(ethylamino)propyl]-1,7-Heptandiamin und einem cytotoxischen Mittel, ausgewählt aus einem antineoplastischen Vincaalkaloid, einem antineoplastischen Antibiotikum, einem antineoplastischen Antimetaboliten und einem antineoplastischen Platinkoordinationskomplex, mit einem pharmazeutisch verträglichen Träger.

2. Verfahren nach Anspruch 1, wobei es sich bei dem cytotoxischen Mittel um Vinblastin handelt.

3. Verfahren nach Anspruch 1, wobei es sich bei dem cytotoxischen Mittel um Cisplatin handelt.

4. Verfahren nach Anspruch 1, wobei es sich bei dem cytotoxischen Mittel um Adriamycin handelt.

5. Verfahren nach Anspruch 1, wobei es sich bei dem cytotoxischen Mittel um Cytarabin handelt.

6. Verfahren nach Anspruch 1, wobei es sich bei dem neoplastischen Krankheitszustand um eine Leukämie handelt.

7. Verfahren nach Anspruch 1, wobei es sich bei dem neoplastischen Krankheitszustand um ein Karzinom handelt.

**Revendications**

**Revendications pour les Etats contractants suivants : AT, BE, CH, DE, DK, FR, GB, IT, LI, LU, NL, SE**

1. Utilisation de N,N'-bis[3-(éthylamino)propyl]-1,7-heptanediamine et d'un agent cytotoxique sélectionné dans le groupe constitué par un alcaloïde de la pervenche antinéoplasique, un antibiotique antinéoplasique, un antimétabolite antinéoplasique et un complexe de coordination du platine antinéoplasique pour la préparation de compositions pharmaceutiques utiles dans le traitement par association chez un malade présentant un tableau clinique néoplasique.

2. Utilisation selon la revendication 1, où l'agent cytotoxique est la vinblastine.

3. Utilisation selon la revendication 1, où l'agent cytotoxique est le cisplatine.

4. Utilisation selon la revendication 1, où l'agent cytotoxique est l'adriamycine.

5. Utilisation selon la revendication 1, où l'agent cytotoxique est la cytarabine.

6. Utilisation selon la revendication 1, où le tableau clinique néoplasique est une leucémie.

7. Utilisation selon la revendication 1, où le tableau clinique néoplasique est un cancer.

**Revendications pour les Etats contractants suivants : ES, GR**

1. Procédé de préparation de compositions pharmaceutiques ou d'un kit utiles dans le traitement par association chez un malade présentant un tableau clinique néoplasique, comprenant la combinaison de N,N'-bis[3-(éthylamino)propyl)-1,7-heptanediamine et d'un agent cytotoxique sélectionné dans le groupe constitué par un alcaloïde de la pervenche antinéoplasique, un antibiotique antinéoplasique, un antimétabolite antinéoplasique et un complexe de coordination du platine antinéoplasique avec un véhicule pharmaceutiquement acceptable.

2. Procédé selon la revendication 1, où l'agent cytotoxique est la vinblastine.

3. Procédé selon la revendication 1, où l'agent cytotoxique est le cisplatine.

4. Procédé selon la revendication 1, où l'agent cytotoxique est l'adriamycine.

5. Procédé selon la revendication 1, où l'agent cytotoxique est la cytarabine.

6. Procédé selon la revendication 1, où le tableau clinique néoplasique est une leucémie.

7. Procédé selon la revendication 1, où le tableau clinique néoplasique est un cancer.